# EUROPÄISCHE PATENTANMELDUNG

(11) **EP 0 709 089 A2**
(43) Veröffentlichungstag der Anmeldung: **01.05.1996**
(21) Anmeldenummer: 95113888.2
(22) Anmeldetag: 05.09.1995
(51) Int. Cl.: A61K 31/08, A61K 31/045, A61K 31/22, A61K 31/225, A61K 31/23, A61K 31/25

(54) **Dermatologische Zubereitungen gegen Superinfektionen mit einem Gehalt an ethoxylierten und/oder propoxylierten Alkoholen und/oder Carbonsäuren**

(30) Priorität: 01.10.1994 DE 4435288
(71) Anmelder: Beiersdorf Aktiengesellschaft, D-20245 Hamburg (DE)
(72) Erfinder: Wolf, Florian, Dr., D-20251 Hamburg (DE); Kahlert, Michael, D-22041 Hamburg (DE)

(57) **Zusammenfassung**

Verwendung einer oder mehrerer Verbindungen, gewählt aus der Gruppe der einfach oder mehrfach ethoxylierten bzw. propoxylierten Alkohole und Fettsäuren, wobei diese Alkohole und Fettsäuren unabhängig voneinander eine oder mehrere Alkohol- bzw. Säurefunktion aufweisen oder auch eine oder mehrere Alkohol- und eine oder mehrere Säurefunktionen in einem Molekül vereinen können, als Wirkprinzip gegen Superinfektionen.

## Beschreibung

Dermatologische Zubereitungen gegen Superinfektionen mit einem Gehalt an ethoxylierten und/oder propoxylierten Alkoholen und/oder Carbonsäuren
Die vorliegende Erfindung betrifft Wirkstoffe und dermatologische Zubereitungen, solche Wirkstoffe enthaltend, gegen Superinfektionen der Haut.

Die gesunde menschliche Haut ist mit einer Vielzahl nichtpathogener Mikroorganismen besiedelt. Diese sogenannte Mikroflora der Haut ist nicht nur unschädlich, sie stellt einen wichtigen Schutz zur Abwehr opportunistischer oder pathogener Keime dar.

Bei bestehendem Primärinfekt, d.h., der normalen Keimbesiedelung der Haut, eintretende Neuinfektion mit hohen Keimzahlen eines oder mehrerer oft physiologischer Erreger, beispielsweise Staphylokokken, oft aber auch unphysiologischer Erreger, beispielsweise Candida albicans, kann bei Zusammentreffen ungünstiger Einflüssen eine "Superinfektion" der befallenen Haut auftreten. Die normale Mikroflora der Haut (oder eines anderen Körperorgans) wird dabei von dem Sekundärerreger regelrecht überwuchert.

Solche Superinfektionen können sich, in Abhängigkeit vom betreffenden Keim, in günstig verlaufenden Fällen in unangenehmen Hauterscheinungen (Juckreiz, unschönes äußeres Erscheinungsbild) äußern. In ungünstig verlaufenden Fällen können sie aber zu großflächiger Zerstörung der Haut führen, im schlimmsten Falle sogar im Tode des Patienten gipfeln.

Superinfektionen der vorab geschilderten Art sind z.B. beim Vollbild von AIDS häufig auftretende Sekundärerkrankungen. An sich - jedenfalls in geringen Keimdichten -unschädliche, aber unter Umständen auch ausgesprochen pathogene Keime überwuchern auf diese Weise die gesunde Hautflora. Bei AIDS allerdings sind auch andere Körperorgane von Superinfektionen betroffen.

Ebenso werden derartige Superinfektionen bei einer Vielzahl dermatologischer Erkrankungen, z.B. atopischem Ekzem, Neurodermitis, Akne, seborrhoischer Dermatitis oder Psoriasis beobachtet. Auch viele medizinische und therapeutische Maßnahmen, z.B die Radio- oder Chemotherapie von Tumorerkrankungen, als Nebenwirkung hervorgerufene, medikamentös induzierte Immunsuppression oder aber systemische Antibiotikabehandlung, ebenso wie externe chemische oder physikalische Einflüsse (z.B. Umweltverschmutzung, Smog), fördern das Auftreten von Superinfektionen der äußeren und inneren Organe, insbesondere der Haut und der Schleimhäute.

Zwar ist es im Einzelfalle ohne weiteres möglich, Superinfektionen mit Antibiotika zu bekämpfen, meistens haben solche Substanzen aber den Nachteil unangenehmer Nebenwirkungen. Oft sind Patienten beispielsweise gegen Penicilline allergisch, weswegen eine entsprechende Behandlung sich in einem solchen Falle verbieten würde.

Ferner haben topisch verabreichte Antibiotika den Nachteil, daß sie die Hautflora nicht nur vom Sekundärerreger befreien, sondern auch die an sich physiologische Hautflora stark beeinträchtigen und der natürliche Heilungsprozeß auf diese Weise wieder gebremst wird.

Aufgabe der vorliegenden Erfindung war, die Nachteile des Standes der Technik zu beseitigen und Substanzen und Zubereitungen, solche Substanzen enthaltend, zur Verfügung zu stellen, durch deren Verwendung Superinfektionen geheilt werden können, wobei die physiologische Hautflora keine nenneswerte Einbußen erleidet.

Darüberhinaus war es Aufgabe der vorliegenden Erfindung, Substanzen und kosmetische bzw. dermatologische Zubereitungen, solche Substanzen enthaltend, zur Verfügung zu stellen, die prophylaktisch gegen Superinfektionen wirken.

Es wurde gefunden, und darin liegt die Lösung all dieser Aufgaben, daß die Verwendung einer oder mehrerer Verbindungen, gewählt aus der Gruppe der einfach oder mehrfach ethoxylierten und/oder propoxylierten Alkohole und Fettsäuren, wobei diese Alkohole und Fettsäuren unabhängig voneinander eine oder mehrere Alkohol- bzw. Säurefunktion aufweisen oder auch eine oder mehrere Alkohol- und eine oder mehrere Säurefunktionen in einem Molekül vereinen können, als Wirkprinzip gegen Superinfektionen, den Nachteilen des Standes der Technik abhilft.

Die erfindungsgemäßen ethoxylierten bzw. propoxylierten Alkohole bzw. Fettsäuren können gesättigte oder ungesättigte organische Reste tragen und verzweigt oder unverzweigt sein. Bevorzugt werden sie aus der Gruppe der Alkanole bzw. Alkylcarbonsäuren mit 5 - 25 Kohlenstoffatomen gewählt.

Ferner ist gegebenenfalls von Vorteil, wenn die bei der Ethoxylierung bzw. Propoxylierung gebildete terminale Hydroxylgruppe ebenfalls verethert bzw. verestert vorliegt.

Besonders bevorzugte ethoxylierte organische Verbindungen werden gewählt aus der Gruppe

R'-(O-CH₂-CH₂)ₙ-O-R

Dabei wird R gewählt aus der Gruppe
H-, C₅ ₋ ₂₅ -Alkyl-, 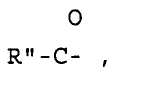
R' wird gewählt aus der Gruppe
C₅₋₂₅-Alkyl-, 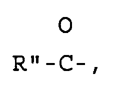
R'' wird gewählt aus der Gruppe C ₅ ₋ ₂₅ -Alkyl-.
n stellt eine Zahl von 1 bis 50 dar.

Besonders bevorzugte propoxylierte organische Verbindungen werden gewählt aus der Gruppe 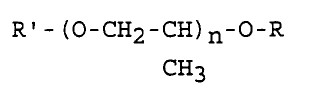
Dabei wird R gewählt aus der Gruppe
C₅ ₋ ₂₅₋ Alkyl-, 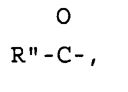
R' wird gewählt aus der Gruppe
C₅ ₋ ₂₅₋ Alkyl-, 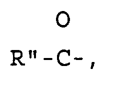
R'' wird gewählt aus der Gruppe C₅ ₋ ₂₅ -Alkyl-.
n stellt eine Zahl von 1 bis 50 dar.

Zwar wird in der DE-OS 42 34 188 die Verwendung der erfindungsgemäß vorteilhaften Substanzen als Antimykotika beschrieben, ihre Verwendung gegen Superinfektionen indes konnte aus dem Stande der Technik nicht hergeleitet werden.

Bevorzugte ethoxylierte bzw. propoxylierte organische Verbindungen im Sinne der vorliegenden Erfindung sind insbesondere Verbindungen, bei welchen R aus der Gruppe H, C₁₂₋₂₀-Alkyl, wobei C₁₈ besonders bevorzugt wird, R' aus der Gruppe C₁₂₋₂₀-Alkyl, wobei C₁₈ besonders bevorzugt wird, gewählt werden und n eine Zahl von 5 - 30 darstellt.

Besonders bevorzugt sind solche ethoxylierte organische Verbindungen, welche aus der Gruppe der nach der CTFA-Nomenklatur Ceteth-X, Ceteareth-X, Steareth-X genannt werden, wobei X eine Zahl von 1 - 50 darstellen kann, bevorzugt von 5 - 30.

Es hat sich in erstaunlicher Weise herausgestellt, daß die erfindungsgemäßen Verbindungen Superinfektionen beseitigen und das natürliche Gleichgewicht der Hautflora wieder herstellen.

Die erfindungsgemäßen Wirkstoffkombinationen sind hervorragend wirksam gegen Superinfektionen, insbesondere gegen Staphylokokken, Pityrosporum ovale sowie Dermatophyten.

Die erfindungsgemäßen Verbindungen werden vorzugsweise in dermatologischen Zusammensetzungen eingesetzt, insbesondere entsprechend einem Gehalt von 0,01 - 50,0 Gew.-%, bevorzugt 0,01 - 20,0 Gew.-%, bezogen auf das Gesamtgewicht der Zusammensetzung. Vorteilhaft enthalten die Zusammensetzungen 0,02 - 10,0 Gew.-%, besonders bevorzugt 0,02 - 5,0 Gew.-% an den erfindungsgemäßen ethoxylierten bzw. propoxylierten organischen Verbindungen, ganz besonders vorteilhaft 0,5 - 3,0 Gew.-%, jeweils bezogen auf das Gesamtgewicht der Zusammensetzung.

Die erfindungsgemäßen Verbindungen lassen sich ohne Schwierigkeiten in gängige dermatologische Formulierungen einarbeiten, vorteilhaft in Pumpsprays, Aerosolsprays, Crèmes, Salben, Tinkturen, Lotionen und dergleichen.

Es ist auch möglich und gegebenenfalls vorteilhaft, die erfindungsgemäßen Verbindungen mit anderen Wirkstoffen zu kombinieren, beispielsweise mit antimikrobiell wirksamen Stoffen.

Zusätzlich zu den genannten Bestandteilen können den erfindungsgemäßen dermatologischen Zubereitungen Parfüm, Farbstoffe, Antioxidantien, Suspendiermittel, Puffergemische oder andere übliche kosmetische oder dermatologische Grundstoffe beigemischt werden.

Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl - und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis µmol/kg), ferner (Metall)-Chelatoren (z.B.α-Hydroxyfettsäuren, Palmitinsäure, Phytinsäure, Lactoferrin), α-Hydroxysäuren (z.B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin A-palmitat) sowie Konyferylbenzoat des Benzoeharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄) Selen und dessen Derivate (z.B. Selenmethionin), Stilbene und deren Derivate (z.B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Es ist vorteilhaft, die erfindungsgemäßen Zusammensetzungen abzupuffern. Vorteilhaft ist ein pH-Bereich von 3,5 - 7,5. Besonders günstig ist es, den pH-Wert in einem Bereich von 4,0 - 6,0 zu wählen.

Die erfindungsgemäßen dermatologischen Formulierungen können wie üblich zusammengesetzt sein und zur Behandlung der Haut im Sinne einer dermatologischen Behandlung dienen.

Zur Anwendung werden die erfindungsgemäßen Formulierungen in der für Dermatika üblichen Weise auf die Haut in ausreichender Menge aufgebracht.

Die erfindungsgemäßen dermatologischen Zubereitungen können Hilfsstoffe enthalten, wie sie üblicherweise in solchen Zubereitungen verwendet werden, z.B. Konservierungsmittel, Bakterizide, Parfüme, Mittel zum Verhindern des Schäumens, Farbstoffe, Pigmente, die eine färbende Wirkung haben, Verdickungsmittel, oberflächenaktive Substanzen, Emulgatoren, weichmachende Substanzen, anfeuchtende bzw. feuchthaltende Substanzen, Fette, Öle, Wachse oder andere übliche Bestandteile einer dermatologischen Formulierung wie Alkohole, Polyole, Polymere, Schaumstabilisatoren, Elektrolyte, organische Lösungsmittel oder Silikonderivate.

Sofern die dermatologische Zubereitung eine Lösung oder Lotion darstellt, können als Lösungsmittel verwendet werden:
- Wasser oder wäßrige Lösungen;
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Gele gemäß der Erfindung enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat wie beispielsweise Bentonite, bei wäßrigalkoholischen oder alkoholischen Gelen vorzugweise ein Polyacrylat ist. Weitere Beispiele für vorteilhafte Verdickungsmittel sind Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel oder ein Gemisch aus Polyethylenglykol und Polyethylenglycolstearat oder -distearat. Das oder die Verdickungsmittel sind in einem Gel z.B. in einer Menge zwischen 0,1 und 30 Gew.-%, bevorzugt zwischen 0,5 und 15 Gew.-%, enthalten.

Feste Stifte gemäß der Erfindung enthalten z.B. natürliche oder synthetische Wachse, Fettalkohole oder Fettsäureester.

Als Treibmittel für erfindungsgemäße, aus Aerosolbehältern versprühbare dermatologische Zubereitungen sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die vorliegende Erfindung geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Bevorzugt können die erfindungsgemäßen Zubereitungen außerdem Substanzen enthalten, die UV-Strahlung absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung. Die nachfolgenden Beispiele sollen die vorliegende Erfindung verdeutlichen, ohne sie einzuschränken.

### Beispiel 1

| | Gew.-% |
|---|---|
| Ceteareth-6 + Stearylalkohol ("Cremophor A 6) | 2,00 |
| Ceteareth-25 ("Cremophor A 25") | 2,00 |
| Cetearyloctanoat ("Luvitol EHO") | 6,00 |
| Cetylstearylalcohol | 0,50 |
| Glycerinmonostearat | 6,00 |
| Paraffinöl DAB 9 | 6,00 |
| Tocopherylacetat | 3,00 |
| 1,2-Propylenglycol | 3,00 |
| Konservierungsmittel, Parfum, Stabilisatoren usw. | nach Belieben |
| Wasser | ad 100,00 |

### Beispiel 2

| | Gew.-% |
|---|---|
| Ceteareth-6 + Stearylalkohol ("Cremophor A 6") | 1,50 |
| Ceteareth-25 ("Cremophor A 25") | 1,50 |
| Cetearyloctanoat ("Luvitol EHO") | 4,00 |
| Glycerinmonostearat | 2,50 |
| Cetylalkohol | 1,50 |
| Paraffinöl DAB 9 | 4,00 |
| 1,2-Propylenglycol | 3,00 |
| Konservierungsmittel, Parfum, Stabilisatoren usw. | nach Belieben |
| Wasser | ad 100,00 |

### Beispiel 3

| Hautschutzcrème | |
|---|---|
| | Gew.-% |
| Ceteareth-6 + Stearylalkohol ("Cremophor A 6") | 2,00 |
| Ceteareth-25 ("Cremophor A 25") | 2,00 |
| Glycerinmonostearat | 5,00 |
| Cetylalkohol | 5,00 |
| Paraffinöl DAB 9 | 9,00 |
| Dimethicone ("Abil 100") | 1,00 |
| Polyquaternium-16 ("Luviquat FC 370") | 5,00 |
| Glycerin | 10,00 |
| Konservierungsmittel, Parfum, Stabilisatoren usw. | nach Belieben |
| Wasser | ad 100,00 |

### Beispiel 4

| | Gew.-% |
|---|---|
| Triceteareth-4 Phosphat ("Hostacerin KW 340 N") | 10,00 |
| Ceteareth-3 ("Hostacerin T-3") | 3,00 |
| Cetylalkohol DAB 7 | 7,00 |
| Isopropylmyristat | 7,00 |
| Paraffinöl DAB 7, dickflüssig | 1,80 |
| GS-Salbenwachs (G.Schütz) | 2,00 |
| Konservierungsmittel, Parfum, Stabilisatoren usw. | nach Belieben |
| Wasser | ad 100,00 |

## Patentansprüche

1. Verwendung einer oder mehrerer Verbindungen, gewählt aus der Gruppe der einfach oder mehrfach ethoxylierten und/oder propoxylierten Alkohole und säuren, wobei diese Alkohole und Fettsäuren unabhängig voneinander eine oder mehrere Alkohol- bzw. Säurefunktion aufweisen oder auch eine oder mehrere Alkohol- und eine oder mehrere Säurefunktionen in einem Molekül vereinen können, als Wirkprinzip gegen Superinfektionen.

2. Verwendung nach Anspruch 1, dadurch gekennzeichnet, daß die bei der Ethoxylierung bzw. Propoxylierung gebildete terminale Hydroxylgruppe ebenfalls verethert bzw. verestert vorliegt.

3. Verwendung nach Anspruch 1, dadurch gekennzeichnet, daß die ethoxylierten organischen Alkohole bzw. Carbonsäuren gewählt werden aus der Gruppe
R'-(O-CH₂-CH₂)ₙ-O-R
Dabei wird R gewählt aus der Gruppe
H-, C₅ ₋ ₂₅ -Alkyl-, 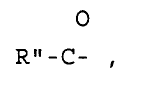 R' wird gewählt aus der Gruppe
C₅₋₂₅-Alkyl-, 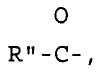 R'' wird gewählt aus der Gruppe C ₅ ₋ ₂₅ -Alkyl-.
n stellt eine Zahl von 1 bis 50 dar.

4. Verwendung nach Anspruch 1, dadurch gekennzeichnet, daß die propoxylierten Alkohole bzw. Carbonsäuren gewählt werden aus der Gruppe 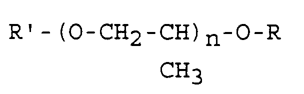 Dabei wird R gewählt aus der Gruppe
C₅ ₋ ₂₅₋ Alkyl-, 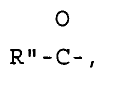 R' wird gewählt aus der Gruppe
C₅ ₋ ₂₅₋ Alkyl-, 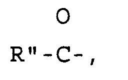 R'' wird gewählt aus der Gruppe C₅ ₋ ₂₅ -Alkyl-.
n stellt eine Zahl von 1 bis 50 dar.

5. Verwendung nach Anspruch 1, dadurch gekennzeichnet, daß die ethoxylierten bzw. propoxylierten Alkohole bzw. Carbonsäuren in dermatologischen Zusammensetzungen in einer Konzentration von 0,01 - 50,0 Gew.-% , vorteilhaft 0,01 - 20,0 Gew.-%, insbesondere 0,01 - 10,0 Gew.-%, besonders bevorzugt 0,02 - 5,0 Gew.-%, ganz besonders vorteilhaft 0,5 - 3,0 Gew.-%, enthalten sind, jeweils bezogen auf das Gesamtgewicht der Zusammensetzung.
